(19) Europäisches Patentamt
European Patent Office
Office européen des brevets

(11) Veröffentlichungsnummer: **0 009 634**
**A1**

(12) # EUROPÄISCHE PATENTANMELDUNG

(21) Anmeldenummer: **79103243.6**

(22) Anmeldetag: **03.09.79**

(51) Int. Cl.³: **C 07 D 231/20**
A 01 N 43/56

(30) Priorität: **09.09.78 DE 2839270**

(43) Veröffentlichungstag der Anmeldung:
**16.04.80 Patentblatt 80/8**

(84) Benannte Vertragsstaaten:
**BE CH DE FR GB IT NL**

(71) Anmelder: **BAYER Aktiengesellschaft**
**Zentralbereich Patente,Marken und Lizenzen Bayerwerk**
**D-5090 Leverkusen 1(DE)**

(72) Erfinder: **Maurer, Fritz, Dr.**
**Roeberstrasse 8**
**D-5600 Wuppertal 1(DE)**

(72) Erfinder: **Riebel, Hans-Jochem, Dr.**
**In der Beek 92**
**D-5600 Wuppertal 1(DE)**

(72) Erfinder: **Schröder, Rolf, Dr.**
**Pahlkestrasse 17**
**D-5600 Wuppertal 1(DE)**

(72) Erfinder: **Hammann, Ingeborg, Dr.**
**Belfortstrasse 9**
**D-5000 Köln 1(DE)**

(72) Erfinder: **Homeyer, Bernhard, Dr.**
**Obere Strasse 28**
**D-5090 Leverkusen 3(DE)**

(72) Erfinder: **Frohberger, Paul-Ernst, Dr.**
**Willi-Baumeister-Strasse 5**
**D-5090 Leverkusen(DE)**

(54) **N,N-Dimethyl-O-pyrazolyl-carbaminsäureester, Verfahren zu ihrer Herstellung und ihre Verwendung als Schädlingsbekämpfungsmittel.**

(57) Die Erfindung betrifft neue N,N-Dimethyl-O-pyrazolylcarbaminsäureester der Formel

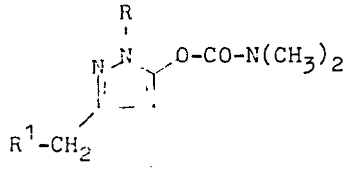

(I)

Verfahren zu ihrer Herstellung sowie ihre Verwendung als Schädlingsbekämpfungsmittel insbesondere als Insektizide, Nematizide und Fungizide.

BAD ORIGINAL

EP 0 009 634 A1

Croydon Printing Company Ltd

0009634



BAYER AKTIENGESELLSCHAFT
Zentralbereich
Patente, Marken und Lizenzen

5090 Leverkusen, Bayerwerk
Ad/Rt-by
Typ Ib

# N,N-Dimethyl-O-pyrazolyl-carbaminsäureester, Verfahren zu ihrer Herstellung und ihre Verwendung als Schädlingsbekämpfungsmittel

Die Erfindung betrifft neue N,N-Dimethyl-O-pyrazolyl-carbaminsäureester, mehrere Verfahren zu ihrer Herstellung sowie ihre Verwendung als Schädlingsbekämpfungsmittel, insbesondere als Insektizide, Nematizide und Fungizide.

Es ist bekannt, daß bestimmte N,N-Dimethyl-O-pyrazolyl-carbaminsäureester, wie z.B. N,N-Dimethyl-O-(1-phenyl-3-methyl-pyrazol(5)yl)- und N,N-Dimethyl-O-(1-iso-propyl-3-methyl-pyrazol(5)yl)-carbaminsäureester, insektizide Eigenschaften aufweisen (vergleiche CH-PS 279 553).

Weiter ist bekannt, daß bestimmte Dithiocarbamate, wie z.B. Zink-äthylen-1,2-bis-(dithiocarbamat), fungizid wirksam sind (vergleich Phytopathology 33 (1943), 1113).

BAD ORIGINAL

Die insektizide, nematizide bzw. fungizide Wirksamkeit dieser aus dem Stand der Technik bekannten Verbindungen ist jedoch, insbesondere bei niedrigen Wirkstoffkonzentrationen und Aufwandmengen, nicht immer zufriedenstellend.

Es wurden nun neue N,N-Dimethyl-O-pyrazolyl-carbaminsäureester der Formel I

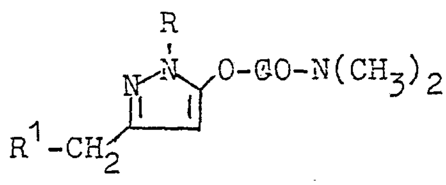

(I)

gefunden, in welcher

R für Cycloalkyl steht und

$R^1$ für Alkoxy, Alkylthio, Alkylsulfinyl oder Alkylsulfonyl steht.

Man erhält die N,N-Dimethyl-O-pyrazolyl-carbaminsäureester der Formel (I), wenn man

a) 5-Hydroxy-pyrazole der Formel II

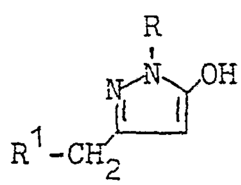

(II)

in welcher R und $R^1$ die oben angegebene Bedeutung haben, mit N,N-Dimethyl-carbaminsäurehalogeniden der Formel III

$$Hal\text{-}CO\text{-}N(CH_3)_2 \qquad (III)$$

in welcher

Hal für Chlor oder Brom steht,

gegebenenfalls in Gegenwart eines Säureakzeptors und gegebenenfalls unter Verwendung eines inerten Verdünnungsmittels umsetzt, oder

b) 5-Hydroxy-pyrazole der Formel (II), in welcher R und $R^1$ die oben angegebene Bedeutung haben,

mit Phosgen und anschließend mit Dimethylamin, gegebenenfalls in Gegenwart eines Säureakzeptors und gegebenenfalls unter Verwendung eines inerten Verdünnungsmittels umsetzt.

Verbindungen der Formel (I), inwelcher R die oben angegebene Bedeutung hat und $R^1$ für Alkylsulfinyl steht, erhält man auch, indem man

c) N,N-Dimethyl-O-(3-alkylthiomethyl-pyrazol(5)yl)-carbaminsäureester der Formel (I), in welcher R die oben angegebene Bedeutung hat und $R^1$ für Alkylthio steht,
mit der äquimolaren Menge Wasserstoffperoxid, gegebenenfalls unter Verwendung eines Verdünnungsmittels umsetzt.

Verbindungen der Formel (I) in welcher R die oben angegebene Bedeutung hat und $R^1$ für Alkylsulfonyl steht, erhält man auch, indem man

d) N,N-Dimethyl-O-(3-alkylthiomethyl-pyrazol(5)yl)-carbaminsäureester der Formel (I), in welcher R die oben angegebene Bedeutung hat und $R^1$ für Alkylthio steht,

mit wenigstens zwei Moläquivalenten m-Chlor-perbenzoesäure, gegebenenfalls in Gegenwart eines Verdünnungsmittels umsetzt.

Die neuen N,N-Dimethyl-O-pyrazolyl-carbaminsäureester der Formel (I) zeichnen sich durch hohe Wirksamkeit als Schädlingsbekämpfungsmittel, insbesondere durch ihre hervorragende insektizide, nematizide und fungizide Wirkung aus.

Überraschenderweise zeigen die erfindungsgemäßen N,N-Dimethyl-O-pyrazolyl-carbaminsäureester eine erheblich höhere insektizide und nematizide Wirkung als die aus dem Stand der Technik vorbekannten Verbindungen analoger Konstitution und gleicher Wirkungsrichtung und eine wesentlich bessere fungizide Wirkung als das bekannte Zink-äthylen-1,2-bis-(dithiocarbamat).

Die Erfindung betrifft vorzugsweise Verbindungen der Formel (I), in welcher

R für Cycloalkyl mit 3 bis 7 Kohlenstoffatomen steht und

$R^1$ für Alkoxy, Alkylthio, Alkylsulfinyl oder Alkylsulfonyl, jeweils mit geradkettigem oder verzweigtem Alkylrest mit 1 bis 5, insbesondere mit 1 bis 3 Kohlenstoffatomen steht.

Verwendet man beispielsweise bei Verfahren (a) N,N-Dimethylcarbaminsäurechlorid, bei Verfahren (b) Phosgen und Dimethylamin und bei beiden Verfahren 1-Cyclopropyl-3-methylsulfonylmethyl-5-hydroxy-pyrazol, bei Verfahren (c) N,N-Dimethyl-O-(1-cyclopentyl-3-iso-propylthiomethyl-pyrazol(5)yl)-carbaminsäureester und Wasserstoffperoxid und bei Verfahren (d) N,N-Dimethyl-O-(1-cyclobutyl-3-äthylthiomethyl-pyrazol(5)yl)-carbaminsäureester und m-Chlor-perbenzoesäure als Ausgangsstoffe, so können die entsprechenden Reaktionen durch folgende Formelschemata skizziert werden:

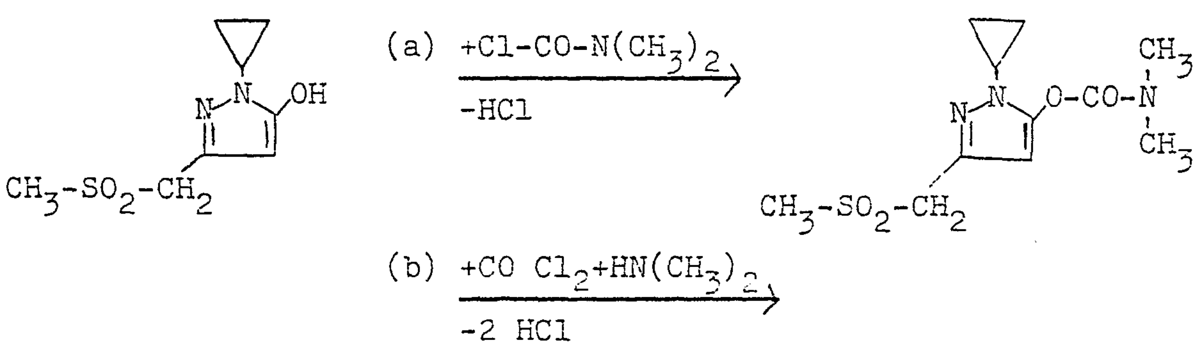

(c)

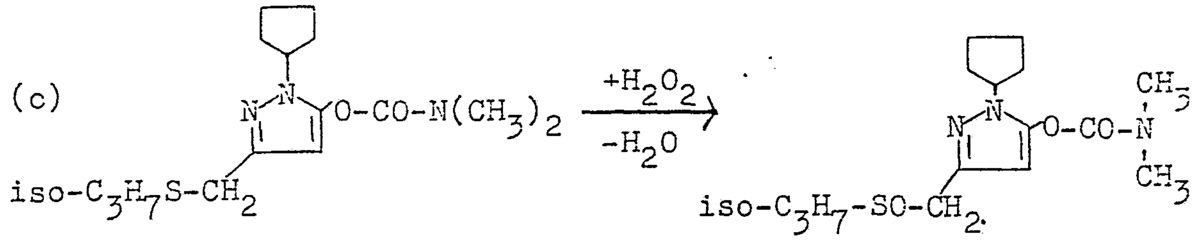


(d)

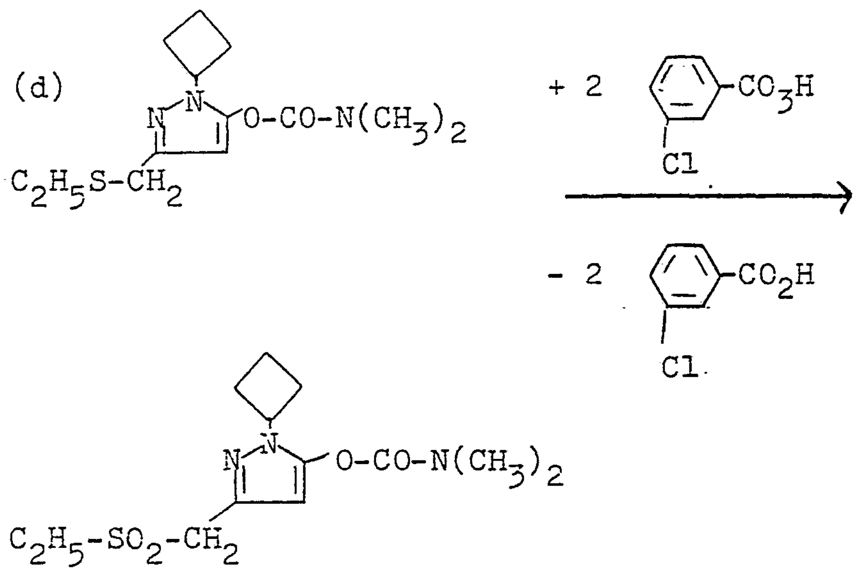

Die bei den Verfahren (a) und (b) als Ausgangsstoffe zu verwendenden 5-Hydroxy-pyrazole sind durch Formel (II) definiert. Vorzugsweise stehen darin R und $R^1$ für diejenigen Reste, welche bei der Definition der Reste R und $R^1$ in Formel (I) als bevorzugt genannt wurden.

Man erhält die 5-Hydroxy-pyrazole der Formel (II) beispielsweise durch Umsetzung von γ-Alkoxy- bzw. γ-Alkylthio-acetessigsäurealkylestern mit Cycloalkylhydrazinen oder deren Hydrochloriden bei Temperaturen zwischen o und 5o°C, gegebenenfalls in Gegenwart eines Säureakzeptors wie z.B. Natriummethylat und unter Verwendung eines Verdünnungsmittels wie z.B. Methanol. Die nach Abdampfen der flüchtigen Komponenten erhaltenen Rohprodukte können

direkt weiter verwendet werden. Die als Vorprodukte benötigten γ-Alkoxy- bzw. γ-Alkylthio-acetessigsäure-alkylester erhält man durch Umsetzung von Acetessigsäurealkylestern mit Halogenierungsmitteln wie z.B. Brom und anschließend mit Alkalialkoholaten bzw. -mercaptiden bei Temperaturen zwischen -10 und +50°C gegebenenfalls in Gegenwart von Verdünnungsmitteln wie z.B. Äther oder Äthanol (vergleiche DE-OS 2 644 588).

Aus den 3-Alkylthiomethyl-5-hydroxy-pyrazolen können analog Verfahren (c) bzw. (d) durch Oxidation mit Wasserstoffperoxid bzw. m-Chlor-perbenzoesäure die entsprechenden 3-Alkylsulfinylmethyl- bzw. 3-Alkylsulfonyl-methyl-5-hydroxy-pyrazole hergestellt werden.

Als Beispiele für die erfindungsgemäß als Ausgangsstoffe zu verwendenden 5-Hydroxy-pyrazole der Formel (II) seien genannt:
3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthio-methyl-, 3-Methylsulfinylmethyl-, 3-Äthylsulfinyl-methyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-1-cyclopropyl-5-hydroxy-pyrazol,
3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-,

3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-1-cyclobutyl-5-hydroxy-pyrazol,
3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-1-cyclopentyl-5-hydroxy-pyrazol,
3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-1-cyclohexyl-5-hydroxy-pyrazol,
3-Methoxymethyl-, 3-Äthoxymethyl-, 3-n-Propoxymethyl-, 3-iso-Propoxymethyl-, 3-Methylthiomethyl-, 3-Äthylthiomethyl-, 3-n-Propylthiomethyl-, 3-iso-Propylthiomethyl-, 3-Methylsulfinylmethyl-, 3-Äthylsulfinylmethyl-, 3-n-Propylsulfinylmethyl-, 3-iso-Propylsulfinylmethyl-, 3-Methylsulfonylmethyl-, 3-Äthylsulfonylmethyl-, 3-n-Propylsulfonylmethyl- und 3-iso-Propylsulfonylmethyl-1-cycloheptyl-5-hydroxy-pyrazol.

Als Beispiel für die bei Verfahren (a) zu verwendenden Carbaminsäurehalogenide der Formel (III) sei N,N-Di-

methylcarbaminsäurechlorid genannt. Es ist, ebenso wie die bei Verfahren (b) einzusetzenden Reaktionskomponenten Phosgen und Dimethylamin, lange bekannt.

Die bei den Verfahren (c) und (d) als Ausgangsstoffe einzusetzenden N,N-Dimethyl-O-(3-alkylthiomethyl-pyrazol(5)yl)-carbaminsäureester sind durch die Formel (I) mit der Maßgabe, daß $R^1$ für Alkylthio steht, definiert. Vorzugsweise stehen darin

R für Cycloalkyl mit 3 bis 7 Kohlenstoffatomen und

$R^1$ für Alkylthio mit 1 bis 5, insbesondere mit 1 bis 3 Kohlenstoffatomen.

Als Beispiele für diese Ausgangsverbindungen seien genannt:
O-(1-Cyclopropyl-3-methylthiomethyl-pyrazol(5)yl)-,
O-(1-Cyclopropyl-3-äthylthiomethyl-pyrazol(5)yl)-,
O-(1-Cyclopropyl-3-n-propylthiomethyl-pyrazol(5)yl)-,
O-(1-Cyclopropyl-3-iso-propylthiomethyl-pyrazol(5)yl)-,
O-(1-Cyclobutyl-3-methylthiomethyl-pyrazol(5)yl-,
O-(1-Cyclobutyl-3-äthylthiomethyl-pyrazol(5)yl)-,
O-(1-Cyclobutyl-3-n-propylthiomethyl-pyrazol(5)yl)-,
O-(1-Cyclobutyl-3-iso-propylthiomethyl-pyrazol(5)yl)-,
O-(1-Cyclopentyl-3-methylthiomethyl-pyrazol(5)yl)-,
O-(1-Cyclopentyl-3-äthylthiomethyl-pyrazol(5)yl)-,
O-(1-Cyclopentyl-3-n-propylthiomethyl-pyrazol(5)yl)-,
O-(1-Cyclopentyl-3-iso-propylthiomethyl-pyrazol(5)yl)-,

O-(1-Cyclohexyl-3-methylthiomethyl-pyrazol(5)yl)-,
O-(1-Cyclohexyl-3-äthylthiomethyl-pyrazol(5)yl)-,
O-(1-Cyclohexyl-3-n-propylthiomethyl-pyrazol(5)yl)-,
O-(1-Cyclohexyl-3-iso-propylthiomethyl-pyrazol(5)yl)-,
O-(1-Cycloheptyl-3-methylthiomethyl-pyrazol(5)yl)-,
O-(1-Cycloheptyl-3-äthylthiomethyl-pyrazol(5)yl)-,
O-(1-Cycloheptyl-3-n-propylthiomethyl-pyrazol(5)yl)-,
O-(1-Cycloheptyl-3-iso-propylthiomethyl-pyrazol(5)yl)-
N,N-dimethyl-carbaminsäureester.

Die in Verfahren (c) bzw. (d) zu verwendenden Oxidationsmittel Wasserstoffperoxid bzw. m-Chlor-perbenzoesäure sind bekannte Verbindungen.

Die Verfahren (a), (b), (c) und (d) zur Herstellung der neuen N,N-Dimethyl-O-pyrazolyl-carbaminsäureester werden bevorzugt unter Verwendung von Verdünnungsmitteln durchgeführt. Als solche kommen praktisch alle inerten organischen Lösungsmittel infrage.

Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Für Verfahren (c) werden aliphatische Carbonsäuren, wie z.B. Essigsäure, als Lösungsmittel bevorzugt.

Verfahren (a) und (b) werden bevorzugt unter Verwendung von Säureakzeptoren durchgeführt.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die erfindungsgemäßen Verfahren werden im allgemeinen bei Temperaturen zwischen 0 und 1oo°C durchgeführt. Bevorzugt wird für Verfahren (a) und (b) der Bereich zwischen 2o und 8o°C, für Verfahren (c) und (d) der Bereich zwischen 0 und 25°C.

Die Umsetzungen werden im allgemeinen bei Normaldruck durchgeführt.

Zur Durchführung von Verfahren (a) und (b) werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt. Nach Reaktionsende gießt man die Mischung in Wasser und schüttelt mit einem organischen Lösungsmittel, z.B. Toluol aus. Die organische Phase wird dann wie üblich durch Waschen, Trocknen und Abdestillieren des Lösungsmittels aufgearbeitet.

Bei Verfahren (c) werden die Reaktionspartner ebenfalls bevorzugt in äquimolaren Mengen eingesetzt. Die in der Regel hierbei als Verdünnungsmittel verwendete Essigsäure wird nach Reaktionsende im Vakuum abdestilliert. Danach gibt man ein organisches Lösungsmittel, z.B. Methylenchlorid zu und arbeitet die organische Phase wie üblich durch Waschen, Trocknen und Abdestillieren des Lösungsmittels auf.

Bei Verfahren (d) wird die als Oxidationsmittel verwendete m-Chlor-perbenzoesäure gewöhnlich im Überschuß eingesetzt, und zwar zwischen 2 und 3 Mol je Mol O-(3-Alkylthiomethyl-pyrazol(5)yl)-N,N-dimethylcarbaminsäureester. Die Umsetzung wird gewöhnlich in einem mit Wasser nicht mischbaren Lösungsmittel durchgeführt. Man wäscht dann neutral und arbeitet wie unter (a) und (c) beschrieben auf.

Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Soweit die neuen Produkte nach dem Abdestillieren des Lösungsmittels in fester Form anfallen, werden sie durch Umkristallisation gereinigt. Zur Charakterisierung dient der Schmelzpunkt.

Die neuen Verbindungen der Formel (I) zeichnen sich, wie bereits erwähnt, durch hervorragende insektizide, nematizide und fungizide Wirksamkeit aus.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer deh Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A

Myzus-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1,2,3,4,5,6,7,8,9,17 und 18

Beispiel B

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1 und 2

Beispiel C

Sproßbehandlungs-Test / Getreiderost / protektiv (blattzerstörende Mykose)

Zur Herstellung einer zweckmäßigen Wirkstoffzubereitung nimmt man 0,25 Gew.-Teile Wirkstoff in 25 Gew.-Teilen Dimethylformamid und 0,06 Gew.-Teilen Emulgator Alkylaryl-polyglykoläther auf und gibt 975 Gew.-Teile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1 %igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100 %igen Luftfeuchtigkeit in ein Gewächshaus.

Nach 10 Tagen Verweilzeit der Pflanzen bei einer Temperatur von 20°C und einer Luftfeuchtigkeit von 80-90 % wertet man den Besatz der Pflanzen mit Rostpusteln aus. Der Befallsgrad wird in Prozenten des Befalls der unbehandelten Kontrollpflanzen ausgedrückt. Dabei bedeutet 0 % keinen Befall und 100 % den gleichen Befallsgrad wie bei der unbehandelten Kontrolle. Der Wirkstoff ist umso wirksamer, je geringer der Rostbefall ist.

Bei diesem Test zeigen z.B. die folgenden Verbindungen der Herstellungsbeispiele überlegene Wirkung gegenüber dem Stand der Technik: 1

Beispiel D

Grenzkonzentrations-Test

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirkung gegenüber dem Stand der Technik: 2,3,4,6,8,9,17 und 18.

## Herstellungsbeispiele

Beispiel 1: 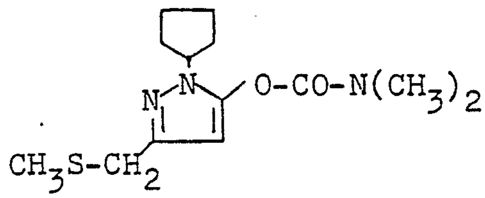

Eine Suspension aus 63,6 g (o,3 Mol) 1-Cyclopentyl-3-methylthiomethyl-5-hydroxy-pyrazol, 62,1 g (o,45 Mol) gemahlener Pottasche und 3oo ml Acetonitril läßt man eine Stunde bei 5o °C rühren, kühlt sie anschließend auf Raumtemperatur ab und fügt 32,3 g (o,3 Mol) N,N-Dimethylcarbaminsäurechlorid hinzu. Nach 7-stündigem Rühren bei 6o °C wird die Reaktionslösung mit 2oo ml Wasser versetzt und mit 3oo ml Toluol ausgeschüttelt. Die organische Phase wird über Magnesiumsulfat getrocknet, filtriert und das Solvens am Rotationsverdampfer im Vakuum abgezogen. Es verbleiben 54 g (64 % der Theorie) N,N-Dimethyl-O-(1-cyclopentyl-3-methylthiomethyl-pyrazol(5)yl)-carbaminsäureester in Form eines braunen Öles mit dem Brechungsindex $n_D^{20}$:1,5348.

Beispiel 2: 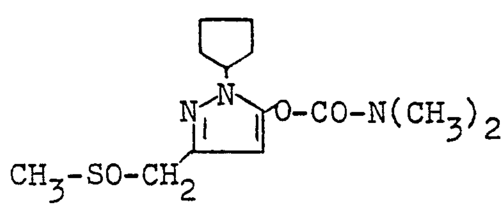

Eine Lösung von 19,8 g (o,o7 Mol) N,N-Dimethyl-O-(1-cyclopentyl-3-methylthiomethyl-pyrazol(5)yl)-carbamin-

säureester in 1oo ml Eisessig wird bei 5-1o°C mit 4,8 g (o,o7 Mol) 5o%igem Wasserstoffperoxid versetzt. Man rührt das Gemisch 6 Stunden bei Raumtemperatur nach und destilliert dann das Lösungsmittel im Vakuum ab. Der Rückstand wird in 1oo ml Methylenchlorid gelöst und mit einer Lösung von 1o g Kaliumcarbonat in 15 ml Wasser gewaschen. Die organische Phase wird abgetrennt und über Natriumsulfat getrocknet. Dann destilliert man das Lösungsmittel im Vakuum ab. Man erhält so 18 g (86 % der Theorie) N,N-Dimethyl-O-(1-cyclopentyl-3-methylsulfinylmethyl-pyrazol(5)yl)-carbaminsäureester in Form eines braunen Öles mit dem Brechungsindex $n_D^{2o}$: 1,52o8.

Beispiel 3:

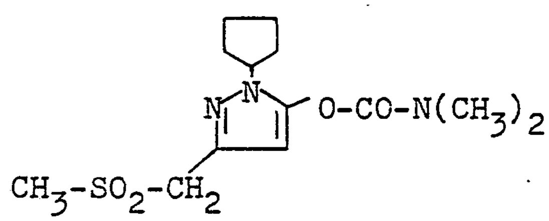

Zu einer Lösung von 14,2 g (o,o5 Mol) N,N-Dimethyl-O-(1-cyclopentyl-3-methylthiomethyl-pyrazol(5)yl)-carbaminsäureester in 5o ml Chloroform tropft man bei 5°C eine Lösung von 21,3 g m-Chlorperbenzoesäure in 15o ml Chloroform. Das Gemisch wird über Nacht bei Raumtemperatur nachgerührt und dann filtriert. Man wäscht das Filtrat mit 1o ml konzentrierter Kaliumcarbonatlösung und trocknet es über Natriumsulfat. Dann wird das Lösungsmittel im Vakuum abgezogen. Zurück bleiben 12,5 g (79,% der Theorie) N,N-Dimethyl-O-(1-cyclopentyl-3-methylsulfonylmethyl-pyrazol(5)yl)-carbaminsäureester in Form eines braunen Öles mit dem Brechungsindex $n_D^{23}$:1,518o.

Analog einem der Beispiele 1 bis 3 können die folgenden Verbindungen der Formel

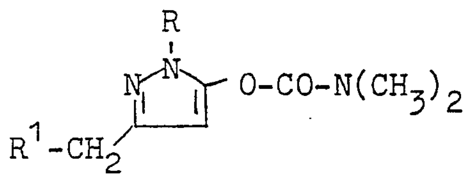

(I)

hergestellt werden:

| Beispiel Nr. | R | $R^1$ | Ausbeute (% der Theorie) | Brechungsindex: |
|---|---|---|---|---|
| 4 | 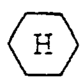 | $SCH_3$ | 95 | $n_D^{23}$:1,5269 |
| 5 | 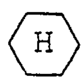 | $SO-CH_3$ | 82 | $n_D^{24}$:1,5241 |
| 6 | 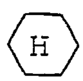 | $SO_2-CH_3$ | 94 | $n_D^{24}$:1,5lo2 |
| 7 | 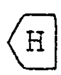 | $SC_2H_5$ | 58 | $n_D^{23}$:1,53oo |
| 8 | 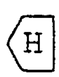 | $SO-C_2H_5$ | 68 | $n_D^{2o}$:1,533o |
| 9 | 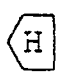 | $SO_2-C_2H_5$ | 5o | $n_D^{2o}$:1,5219 |
| lo | 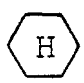 | $SC_2H_5$ | | |
| 11 | 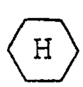 | $SO_2-C_2H_5$ | | |
| 12 | 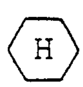 | $SO-C_2H_5$ | | |

| Beispiel Nr. | R | $R^1$ | Ausbeute (% der Theorie) | Brechungsindex: |
|---|---|---|---|---|
| 13 | 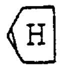 | $SC_3H_7$-n | | |
| 14 | 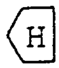 | $SC_3H_7$-iso | | |
| 15 | 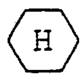 | $SC_3H_7$-n | | |
| 16 | 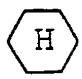 | $SC_3H_7$-iso | | |
| 17 | 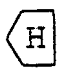 | $OCH_3$ | 72 | $n_D^{20}$:1,4976 |
| 18 | 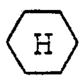 | $OCH_3$ | 78 | $n_D^{20}$:1,5123 |
| 19 | 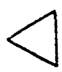 | $SCH_3$ | | |
| 2o | 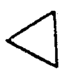 | $SO-CH_3$ | | |
| 21 | 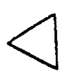 | $SO_2-CH_3$ | | |
| 22 | 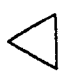 | $OCH_3$ | | |
| 23 | 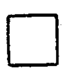 | $SCH_3$ | | |
| 24 | 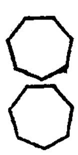 | $SCH_3$ | | |
| 25 | | $SO-CH_3$ | | |
| 26 | 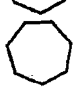 | $SO_2-CH_3$ | | |

Die als Ausgangsmaterialien zu verwendenden substituierten 5-Hydroxypyrazole können zum Beispiel wie folgt hergestellt werden:

Beispiel a:

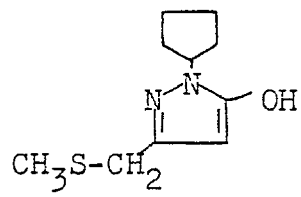

Zu einer Lösung von 15 g (o,11 Mol) Cyclopentylhydrazin-Hydrochlorid in 3o ml Methanol gibt man unter Kühlen o,11 Mol einer Lösung von Natriummethylat in Methanol. Dann versetzt man das Gemisch bei Raumtemperatur mit 17,6 g (o,1 Mol) γ-Methylthioacetessigsäureäthylester und rührt 6 Stunden nach. Anschließend wird das Lösungsmittel im Vakuum abdestilliert, der Rückstand mit Wasser verrieben und nach Kristallisation abgesaugt. Man erhält so 2o g (94 % der Theorie) 1-Cyclopentyl-3-methylthiomethyl-5-hydroxypyrazol in Form beiger Kristalle mit dem Schmelzpunkt 99°C.

In analoger Weise können die folgenden Verbindungen der Formel

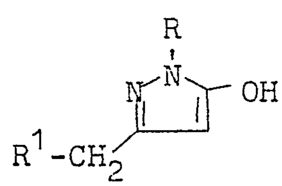

hergestellt werden:

| Beispiel | R | $R^1$ | Ausbeute (% der Theorie) | Schmelzpunkt (°C) |
|---|---|---|---|---|
| b | 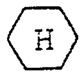 | $SCH_3$ | 95 | 117 |
| c | 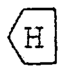 | $SC_2H_5$ | 87 | 9o |
| d | 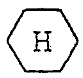 | $SC_2H_5$ | | |
| e | 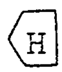 | $SC_3H_7$-n | | |
| f | 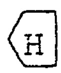 | $SC_3H_7$-iso | | |
| g | 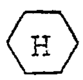 | $SC_3H_7$-n | | |
| h | 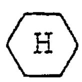 | $SC_3H_7$-iso | | |
| i | 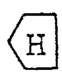 | $OCH_3$ | 68 | ölig |
| j | 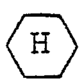 | $OCH_3$ | 8o | ölig |
| k | 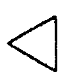 | $SCH_3$ | | |
| l | 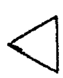 | $OCH_3$ | | |
| m | 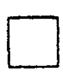 | $SCH_3$ | | |
| n | 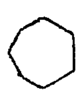 | $SCH_3$ | | |

Patentansprüche

1. N,N-Dimethyl-O-pyrazolyl-carbaminsäureester der Formel (I)

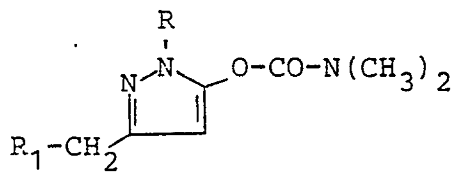

(I)

worin

R für Cycloalkyl steht und

$R^1$ Alkoxy, Alkylthio, Alkylsulfinyl oder Alkylsulfonyl bedeutet.

2. Verfahren zur Herstellung von N,N-Dimethyl-O-pyrazolyl-carbaminsäureestern der Formel (I), dadurch gekennzeichnet, daß man

a) 5-Hydroxy-pyrazole der Formel II

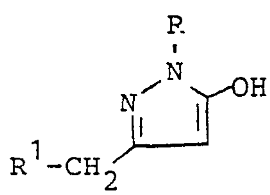

(II)

in welcher

R und $R^1$ die oben angegebene Bedeutung haben,

mit N,N-Dimethyl-carbaminsäurehalogeniden der Formel (III)

$$Hal-Co-N(CH_3)_2 \qquad (III)$$

in welcher

Hal für Chlor oder Brom steht,

gegebenenfalls in Gegenwart eines Säureakzeptors und gegebenenfalls unter Verwendung eines inerten Verdünnungsmittels umsetzt, oder

b) 5-Hydroxy-pyrazole der Formel (II)

in welcher

R und $R^1$ die oben angegebene Bedeutung haben,

mit Phosgen und anschließend mit Dimethylamin, gegebenenfalls in Gegenwart eines Säureakzeptors und gegebenenfalls unter Verwendung eines inerten Verdünnungsmittels umsetzt.

3. Verfahren zur Herstellung von N,N-Dimethyl-O-pyrazolyl-carbaminsäureestern der Formel (I), in welcher R die oben angegebene Bedeutung hat und $R^1$ für Alkylsulfinyl steht, dadurch gekennzeichnet, daß man N,N-Dimethyl-O-(3-alkylthiomethyl-pyrazol(5)yl)-carbaminsäureester der Formel (I), in welcher R die oben angegebene Bedeutung hat und $R^1$ für Alkylthio steht, mit der äquimolaren Menge Wasserstoffperoxid, gegebenenfalls unter Verwendung eines Verdünnungsmittels umsetzt.

4. Verfahren zur Herstellung von N,N-Dimethyl-O-pyrazolyl-carbaminsäureestern der Formel (I) in welcher R die oben angegebene Bedeutung hat und $R^1$ für Alkylsulfonyl steht, dadurch gekennzeichnet, daß man N,N-Dimethyl-O-(3-alkylthiomethyl-pyrazol(5)yl)-carbaminsäureester der Formel (I), in welcher R die oben angegebene Bedeutung hat und $R^1$ für Alkylthio steht, mit wenigstens zwei Moläquivalenten m-Chlor-perbenzoesäure, gegebenenfalls in Gegenwart eines Verdünnungsmittels umsetzt.

5. Schädlingsbekämpfungsmittel gekennzeichnet durch einen Gehalt an mindestens einem N,N-Dimethyl-O-pyrazolyl-carbaminsäureester der Formel (I).

6. Verfahren zur Bekämpfung von Schädlingen, dadurch gekennzeichnet, daß man N,N-Dimethyl-O-pyrazolyl-carbaminsäureester der Formel (I) auf die Schädlinge oder ihren Lebensraum einwirken läßt.

7. Verwendung von N,N-Dimethyl-O-pyrazolyl-carbaminsäureestern der Formel (I) zur Bekämpfung von Schädlingen.

8. Verfahren zur Herstellung von Schädlingsbekämpfungsmitteln, dadurch gekennzeichnet, daß man N,N-Dimethyl-O-pyrazolyl-carbaminsäureester der Formel (I) mit Streckmitteln und/oder oberflächenaktiven Mitteln vermischt.

Europäisches Patentamt

# EUROPÄISCHER RECHERCHENBERICHT

0009634

Nummer der Anmeldung

EP 79 10 3243

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl. 3) |
|---|---|---|---|
| | DE - A - 1 542 661 (AGRIPAT)<br>* Patentansprüche 1-16 *<br>-- | 1,6,7,<br>8 | C 07 D 231/20<br>A 01 N 43/56 |
| | DE - A - 2 644 588 (BAYER)<br>* Patentansprüche, Seiten 30-31 *<br>-- | 1,2,<br>6-8 | |
| P | DE - A - 2 721 188 (BAYER)<br>* Patentansprüche, Seiten 35-36 *<br>---- | 1,2,6,<br>7,8 | |

RECHERCHIERTE SACHGEBIETE (Int. Cl. 3)

C 07 D 231/20

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt

| Recherchenort | Abschlußdatum der Recherche | Prüfer |
|---|---|---|
| Den Haag | 01-11-1979 | CREMERS |